# EUROPÄISCHE PATENTSCHRIFT

(11) **EP 1 032 553 B1**
(45) Veröffentlichungstag und Bekanntmachung des Hinweises auf die Patenterteilung: **29.01.2003**
(21) Anmeldenummer: 98961164.5
(22) Anmeldetag: 09.11.1998
(51) Int. Cl.: C07C 29/149, C07C 33/02, C07C 33/025, C07C 67/54, C07C 69/24, C07C 69/533

(54) **Verfahren zur Herstellung ungesättigter Kokos- und/oder Palmkernfettalkohole**
Process for the preparation of unsaturated coconut and/or palm kernel fatty alcohols
Procédé de préparation d'alcools gras insaturés de noix de coco et/ou de noix de palmier

(30) Priorität: 17.11.1997 DE 19750801
(43) Veröffentlichungstag der Anmeldung: 06.09.2000
(73) Patentinhaber: Cognis Deutschland GmbH & Co. KG, 40589 Düsseldorf (DE)
(72) Erfinder: HECK, Stephan, D-50259 Pulheim (DE); KLEIN, Norbert, D-40822 Mettmann (DE); KOMP, Horst-Dieter, D-40764 Langenfeld (DE); BÖHR, Christiane, D-51379 Leverkusen (DE); HÜBNER, Norbert, D-40597 Düsseldorf (DE); WESTFECHTEL, Alfred, D-40724 Hilden (DE)
(86) Internationale Anmeldenummer: EP9807150
(87) Internationale Veröffentlichungsnummer: WO99025674

(56) Entgegenhaltungen:
- DE-A- 4 335 781
- DE-A- 4 425 180

## Beschreibung

### Gebiet der Erfindung

Die Erfindung betrifft ungesättigte Fettalkohole, die man erhält, indem man Kokos- oder Palmkemfettsäuremethylester einer zweifachen Fraktionierung unterwirft und dann hydriert sowie ein Verfahren zur Herstellung dieser Fettalkohole.

### Stand der Technik

Ungesättigte Fettalkohole stellen wichtige Zwischenprodukte für eine große Anzahl von Erzeugnissen der chemischen Industrie, wie z.B. für die Herstellung von Tensiden und Hauptpflegeprodukten dar. Eine Übersicht hierzu findet sich beispielsweise von U.Ploog et al. in **Seifen-Öle-Fette-Wachse 109, 225 (1983).** Zu ihrer Herstellung geht man von mehr oder minder ungesättigten Fettsäuremethylestern aus, die beispielsweise in Gegenwart chrom- oder zinkhaltiger Mischkatalysatoren hydriert werden können **[Ullmann's Enzyclopaedie der technischen Chemie, Verlag Chemie, Weinheim, 4. Aufl., Bd. 11, S. 436f].** Stand der Technik ist ein großtechnisches Verfahren, wie es bislang auch bei der Anmelderin durchgeführt worden ist, nach dem tierische Fette und Öle eingesetzt werden, die nach der Hydrierung anfallenden ungesättigten Fettalkohole bei einer Sumpftemperatur von z. B. 220 bis 250°C und einem verminderten Druck von 1 bis 20 mbar - gemessen am Kolonnenkopf - destilliert werden. Da die Herstellung von ungesättigten Fettalkoholen mit hohen Kosten verbunden ist, wurde auf einen möglichst geringen Rohstoffverlust destilliert. Tatsächlich konnte auf diese Weise eine Ausbeute von ca. 90 % der Theorie - und entsprechend ein Verlust von 10 % - erreicht werden, die Produkte zeigten jedoch einen deutlichen Eigengeruch. Ein weiterer Nachteil besteht ferner darin, daß die Fettalkohole des Standes der Technik ein unbefriedigendes Lager- und Kälteverhalten zeigen.

Aus anwendungstechnischen Gründen sind ungesättigte Fettalkohole mit lodzahlen von 50 bis 80 besonders bevorzugt, da diese einen für die Verwendung in Kosmetikprodukten günstigen Erstarrungspunkt besitzen. Ungesättigte Fettalkohole mit lodzahlen im oben genannten Bereich basieren derzeit ganz überwiegend auf tierischen Rohstoffen. Der gewünschte lodzahlbereich wird durch Verschneiden verschiedener Produkte mit unterschiedlichen lodzahlbereichen eingestellt. Die Einstellung des lodzahlbereichs durch destillative Verfahren ist nicht möglich, da die lodzahl bzw. der lodzahlbereich von Fettalkoholen bzw. Fettsäuren auf tierischer Basis bei der Fraktionierung nahezu konstant bleibt. Tierische Fette haben aber den Nachteil, daß sie sehr heterogen aufgebaut sind. Zum Beispiel enthalten tierische Fette stickstoffhaltige Verbindungen wie Amide oder Steroide, wie z. B. Cholesterin, die direkt oder indirekt für den oben erwähnten unangenehmen Geruch der Produkte verantwortlich sind. Die stickstoffhaltigen Verbindungen können Nebenreaktionen eingehen, was die Produktstabilität, insbesondere die Oxidationsstabilität, verschlechtert und zu verfärbten Produkten führt. Zudem werden vom Verbraucher im Hinblick auf die andauernde BSE-Diskussion Produkte, die unter Verwendung von Rindertalg hergestellt werden äußerst kritisch gesehen. Auf dem Kosmetikmarkt besteht daher ein stetiges Bedürfnis nach immer reineren und qualitativ hochwertigeren Rohstoffen, eine Forderung, die üblicherweise nur durch immer aufwendigere technische Verfahren und zusätzliche Reinigungsschritte zur Verfügung gestellt werden können. Im Fall der ungesättigten Fettalkohole besteht dabei insbesondere das Bedürfnis nach Produkten mit verbesserter Farb- und Geruchsqualität sowie vorteilhafterem Kälteverhalten. Hinzukommt, daß sich in den letzten Jahren das Verbraucherverhalten dahingehend verändert hat, daß die Verbraucher sehr großen Wert auf rein pflanzliche Produkte legen. Die bekannten pflanzlichen Fettalkohole weisen lodzahlen in dem Bereich unter 20 oder sehr hohe lodzahlen über 100 auf. Fettalkohole mit lodzahlen in dem oben genannten anwendungstechnisch besonders bevorzugten Bereich zwischen 20 und 95 sind nicht bekannt. Das Verschneiden der Fettalkohole mit sehr unterschiedlichen lodzahlen führt nicht zu zufriedenstellenden Produkten. Aus der deutschen Offenlegungsschrift **DE-A1 4335781** (Henkel) ist ein Verfahren bekannt, bei dem man die in den pflanzlichen Fetten Rohstoffen enthaltenen Triglyceride zunächst durch Druckspaltung in Glycerin und Fettsäuren spaltet und letztere mit Methanol verestert bzw. die Ausgangsstoffe direkt zu den Fettsäuremethylestern umestert und anschließend die Ester zu den Alkoholen hydriert, wobei entweder die Fettsäuremethylester oder die Hydrierprodukte durch Abnahme einer solchen Menge Vorlauf fraktioniert werden, daß das Endprodukt eine lodzahl von 20 bis 110 und einen Konjuengehalt von weniger als 4,5 Gew.-% aufweist. Während sich das Verfahren auf pflanzliche Rohstoffe wie Palmkernöl oder Kokosöl für die Herstellung von ungesättigten Fettalkoholen im lodzahlbereich 50 bis 65 problemlos anwenden läßt, werden beim Einsatz der genannten Rohstoffe zur Erzeugung von ungesättigten Fettalkoholen im lodzahlbereich von 65 bis 85 überraschenderweise nicht in jeder Hinsicht befriedigende Ergebnisse erzielt.

Die Aufgabe der vorliegenden Erfindung hat folglich darin bestanden, ungesättigte Fettalkohole auf Basis von Kokos- und/oder Palmkernöl zur Verfügung zu stellen, die lodzahlen im Bereich von 65 bis 85 aufweisen und gegenüber ungesättigten Fettalkoholen auf tierischer Basis eine größere Oxidationsstabilität und ein gleichwertiges oder besseres Kälteverhalten aufweisen. Gleichzeitig sollten möglichst reine Koppelprodukte erhalten werden.

Der Gegenstand der Erfindung betrifft ein Verfahren zur Herstellung ungesättigter Kokos- und Palmkernfettalkohole mit einer lodzahl im Bereich von 65 bis 85, die im wesentlichen ungesättigte Fettalkohole und Gemische aus gesättigten Fettalkoholen der Formel **(I)** enthalten,

**R**^{**1**}**OH (I)**

in der R¹ für einen gesättigten oder ungesättigten, linearen oder verzweigten Alkylrest mit 14 bis 20 Kohlenstoffatomen steht, bei dem man
(a) Kokos- und/oder Palmkernfettsäuremethylester unter Erhalt einer Fraktion mit überwiegend C₁₆-C₁₈-Anteilen fraktioniert,
(b) die C₁₆-C₁₈-Fettsäuremethylesterfraktion in ein überwiegend gesättigtes Destillat und ein überwiegend ungesättigtes Sumpfprodukt fraktioniert, und
(c) das Sumpfprodukt unter Erhalt der Doppelbindungen zu den entsprechenden Alkoholen hydriert.

### 1. Fraktionierung

Die Fraktionierung der Kokos- bzw. Palmkernfettsäuremethylester kann batchweise oder kontinuierlich bei verringertem Druck durchgeführt werden. Die Beheizung kann beispielsweise durch Heißdampf erfolgen, wobei in der Regel eine Sumpftemperatur von z. B. 220 bis 250°C herrscht. Die eigentliche Fraktionierung findet in einer gepackten Kolonne mit druckverlustarmen Einbauten statt. Als Einbauten kommen beispielsweise geordnete Blechpackungen in Betracht. Weitere Beispiele finden sich in **RÖMPP Chemie Lexikon, Thieme Verlag, Stuttgart, 9. Auflage, Bd. 3, S. 2305 (1990)** unter dem Stichwort "Kolonnen-Einbauten" und in der dort genannten Literatur. Das erforderliche Feinvakuum von 1 bis 20 mbar am Kopf der Kolonne kann beispielsweise mit Hilfe von Wasserringpumpen und vorgeschalteten Dampfstrahlern erzielt werden. Vorzugsweise sollte der Druckabfall über die gesamte Destillationsanlage nicht mehr als 20 mbar betragen. Hierbei wird eine Fraktion mit überwiegend C₈-C₁₀-Anteilen, eine weitere Fraktion mit überwiegend C₁₂-C₁₄-Anteilen und schließlich eine dritte Fraktion mit überwiegend C₁₆-C₁₈-Anteilen erhalten; bei letzterer kann es sich um das Sumpfprodukt handeln. Das Gewichtsverhältnis von Destillat und Sumpfprodukt liegt im Bereich von 30 : 70 bis 35 : 65.

### 2. Fraktionierung

Nachdem in der ersten Fraktionierung eine C₁₆-C₁₈-Fettsäureesterfraktion hergestellt wurde, die man auch als Nachlauf bezeichnet, wird diese in der zweiten Fraktionierung in ein weitgehend gesättigtes Destillat, das überwiegend Cetylalkohol enthält, und ein weitgehend ungesättigtes Sumpfprodukt aufgetrennt, das überwiegend aus Oleylalkohol besteht und eine lodzahl im Bereich von 65 bis 85, vorzugsweise 70 bis 75 besitzt. Die zweite Fraktionierung erfolgt unter vergleichbaren Bedingungen wie die erste, allerdings arbeitet man bei etwa 220 bis 250°C und einem verminderten Druck von 5 bis 10 mbar. Das Gewichtsverhältnis von Destillat und Sumpfprodukt liegt im Bereich von 10 : 90 bis 15 : 85.

### Hydrierung

Die abschließende Hydrierung der Methylesterfraktion unter Erhalt der Doppelbindungen kann in an sich bekannter Weise durchgeführt werden, d.h. beispielsweise in Gegenwart von handelsüblichen Zink/Chrom-Katalysatoren, bei Temperaturen im Bereich von 250 bis 350°C sowie einem Wasserstoffdruck von 200 bis 275 bar. Der Konjuengehalt der Produkte liegt dabei im Bereich von 4 bis 12, vorzugsweise 4,5 bis 6 Gew.-%, der Gehalt an Kohlenwasserstoffen unter 3, vorzugsweise unter 1 Gew.-%.

### Gewerbliche Anwendbarkeit

Die nach dem erfindungsgemäßen ungesättigten Kokos- und/oder Palmkernfettalkohole sind farb- und geruchsarm und weisen ein besonders vorteilhaftes Kälteverhalten auf. Sie eignen sich daher als Rohstoffe zur Herstellung von Wasch-, Spül- und Reinigungsprodukten sowie Produkten zur Haar- und Körperpflege, in denen sie in Mengen von 1 bis 50, vorzugsweise 5 bis 30 Gew.-% - bezogen auf die Mittel - enthalten sein können.

### Beispiel

Ein technischer C₈-C₁₈-Kokos/Palmkernfettsäuremethylester wurde in einer gepackten Kolonne mit druckverlustarmen Einbauten bei einer Sumpftemperatur von 200°C und einem Kopfvakuum von 20 mbar fraktioniert. Die beiden kürzerkettigen Fraktionen mit den C₈-C₁₀ und C₁₂-C₁₄-Anteilen wurden abgenommen und getrennt weiterverarbeitet. Die als Sumpf verbliebene C₁₆-C₁₈-Fraktion wurde in einer zweiten Kolonne bei einer Sumpftemperatur von 250°C und einem Kopfvakuum von 5 mbar ein zweitesmal fraktioniert, wobei als Destillat 15 Gew.-% Palmitinsäuremethylester anfielen, während im Sumpf 85 Gew.-% eines C₁₆-C₁₈-Fettsäuremethylestergemisches verblieb, welches eine lodzahl von 72 aufwies. Das Estergemisch wurde in einen Autoklaven überführt und dort in Gegenwart von handelsüblichen Zink/Chrom-Katalysatoren bei 300°C und 250 bar mit Wasserstoff zum Gemisch der korrespondierenden Fettalkohole reduziert. Das von Methanol befreite Hydrierprodukt wies nach gaschromatographischer und naßchemischer Analyse die Kenndaten gemäß Tabelle 1 auf:

**Tabelle 1:**

| Kenndaten Hydrierprodukt | | | |
|---|---|---|---|
| **Zusammensetzung** | **Anteil [Flächen-%]** | **Kenndaten** | **Wert** |
| Cetylalkohol | 15,9 | lodzahl | 72 |
| Palmoleylalkohol | 0,5 | Hydroxylzahl | 211 |
| Margarinylalkohol | 0,4 | Säurezahl | 0,02 |
| Stearylalkohol | 10,7 | Verseifungszahl | 0,3 |
| Oleylalkohol | 62,3 | Hazenfarbzahl | 10 |
| Linolylalkohol | 3,5 | Konjuengehalt | 4,6 Gew.-% |
| Linolenylalkohol | 4,6 | Erstarrungspunkt | 24°C |
| Kohlenwasserstoffe | 2,1 | | |

## Patentansprüche

1. Verfahren zur Herstellung von ungesättigten Kokos- und/oder Palmkernfettalkoholen mit einer Iodzahl im Bereich von 65 bis 85, die im wesentlichen ungesättigte Fettalkohole und Gemische aus gesättigten Fettalkoholen der Formel **(I)** enthalten,
**R**^{**1**}**OH (I)**
in der R¹ für einen gesättigten oder ungesättigten, linearen oder verzweigten Alkylrest mit 14 bis 20 Kohlenstoffatomen steht, bei dem man
(a) Kokos- und/oder Palmkernfettsäuremethylester unter Erhalt einer Fraktion mit überwiegend C₁₆-C₁₈-Anteilen fraktioniert,
(b) die C₁₆-C₁₈-Fettsäuremethylesterfraktion in ein überwiegend gesättigtes Destillat und ein überwiegend ungesättigtes Sumpfprodukt fraktioniert, und
(c) das Sumpfprodukt unter Erhalt der Doppelbindungen zu den entsprechenden Alkoholen hydriert.

## Claims

1. A process for the preparation of unsaturated coconut and/or palm nut fatty alcohols having an iodine number in the range from 65 to 85, which essentially comprise unsaturated fatty alcohols and mixtures of saturated fatty alcohols of the formula **(I)**
**R**^{**1**}**OH (I)** (I)
in which R¹ is a saturated or unsaturated, linear or branched alkyl radical having from 14 to 20 carbon atoms, which comprises
(a) fractionating coconut and/or palm nut fatty acid methyl esters to obtain a fraction containing predominantly C₁₆-C₁₈ portions,
(b) fractionating the C₁₆-C₁₈ fatty acid methyl ester fractions into a predominantly saturated distillate and a predominantly unsaturated bottom product, and
(c) hydrogenating the bottom product with retention of the double bonds to give the corresponding alcohols.

## Revendications

1. Procédé de production d'alcools gras de coco et/ou de palmiste non saturé ayant un indice d'iode dans la zone de 65 à 85, qui contiennent essentiellement des alcools gras non saturés et des mélanges à base d'alcools gras saturés de formule (I)
R¹OH (I)
dans laquelle R¹ représente un reste alkyle saturé ou non saturé, linéaire ou ramifié, ayant de 14 à 20 atomes de carbone, dans lequel
a) on fractionne l'ester méthylique d'acides gras de coco et/ou de palmiste tout en conservant une fraction avec principalement des fractions en C₁₆ - C₁₈.
b) on fractionne la fraction ester méthylique d'acides gras en C₁₆ - C₁₈ en un distillat principalement saturé et un produit de pot principalement non saturé, et
c) on hydrogène le produit de pot tout en conservant les doubles liaisons, en alcools correspondants.
